# EUROPÄISCHE PATENTSCHRIFT

(11) **EP 1 741 395 B1**
(45) Veröffentlichungstag und Bekanntmachung des Hinweises auf die Patenterteilung: **17.02.2010**
(21) Anmeldenummer: 06019502.1
(22) Anmeldetag: 04.03.2002
(51) Int. Cl.: A61B 17/68, A61B 17/92

(54) **Vorrichtung zum Befestigen eines selbsthaltenden Implantats**
Device for attaching a self-retaining implant
Dispositif pour montage d'un implant auto-rétentif

(30) Priorität: 24.04.2001 DE 20107039 U; 01.08.2001 US 921233
(43) Veröffentlichungstag der Anmeldung: 10.01.2007
(62) Teilanmeldung aus: 02004363.4
(73) Patentinhaber: Stryker Leibinger GmbH & Co. KG, 79111 Freiburg (DE)
(72) Erfinder: Knöpfle, Christian, 78166 Donaueschingen (DE); Frank, Thorsten, 78567 Fridingen (DE); Greiner, Karl, 78570 Mühlheim (DE); Eckerle, Hans-Urs, 79117 Freiburg (DE)
(74) Vertreter: Röthinger, Rainer

(56) Entgegenhaltungen:
- US-A- 5 258 010
- US-A- 5 741 268
- US-A- 5 964 762
- US-A- 6 010 513
- US-B1- 6 168 596
- US-B1- 6 190 389

## Beschreibung

Die Offenbarung betrifft ein Implantat zur Festlegung eines Knochendeckels oder Knochenfragments, insbesondere zur Festlegung eines craniotomierten Knochendeckels oder eines Schädelknochenfragments an der Schädelkalotte. Die Erfindung betrifft eine Vorrichtung zum Befestigen dieses Implantats an dem festzulegenden Knochendeckel oder Knochenfragment.

Im Rahmen von Schädeloperationen werden häufig Knochendeckel, d.h. plattenförmige Knochenteile, aus dem Schädelknochen herausgesägt. Diese Knochendeckel müssen nach Abschluss der Operation wieder in der entstandenen Öffnung der Schädeldecke fixiert werden, damit die Knochendeckel in ihrer alten Position wieder einheilen können. Eine vergleichbare Problemstellung tritt auf, wenn Schädelknochenfragmente im Bereich der Schädeldecke fixiert werden müssen.

Zur Festlegung von Knochendeckeln oder Schädelknochenfragmenten wurden unterschiedliche Implantate vorgeschlagen. So sind Implantate bekannt, welche zwei , Schraubenlöcher aufweisen. In einem ersten Schritt werden derartige Implantate mittels Schrauben z.B. an dem festzulegenden Knochendeckel befestigt. Anschließend werden die mit dem Knochendeckel verschraubten Implantate zusätzlich am Schädelknochen festgeschraubt. Aufgrund der Tatsache, dass jedes einzelne Implantat mittels je einer Schraube zunächst am Knochendeckel und anschließend am Schädelknochen befestigt werden muss, ist das Festlegen des Knochendeckels sehr zeitaufwendig. Um den mit der Festlegung eines Knochendeckels verbundenen Zeitaufwand zu reduzieren, wurden selbsthaltende Implantate vorgeschlagen.

Ein derartiges, selbsthaltendes Implantat ist beispielsweise aus der DE 199 07 354 A1 bekannt. Bei diesem Implantat handelt es sich um ein klammerartiges Element, welches auf den festzulegenden Knochendeckel aufgesteckt wird. Das Implantat umfasst hierzu zwei beabstandet voneinander angeordnete Anlegearme, welche im aufgesteckten Zustand des Implantats den Knochendeckel umgreifen. Ein erster der beiden Anlegearme kontaktiert dabei die Oberseite des Knochendeckels und ein zweiter der beiden Anlegearme kontaktiert die Unterseite.

Nachteilig bei einem derartigen, klammerartigen Implantat ist dessen umständliche Handhabung. Die umständliche Handhabung ist in erster Linie darauf zurückzuführen, dass unterschiedliche Knochendeckel nicht immer die selbe Stärke aufweisen und auch die Stärke ein und desselben Knochendeckels variieren kann. Nachteilig ist weiterhin, dass der auf der Unterseite des Knochendeckels anliegende Anlegearm bei festgelegtem Knochendeckel auf den Hirnhautlappen aufliegt und diese unter Umständen verletzt.

Aus der US 5,741,268 ist eine Applikationsvorrichtung bekannt, mit der Membranen im Rahmen eines zahnchirurgischen Eingriffs mit Hilfe eines Betätigungsmechanismus für ein Schlagelement und einer separat bedienbaren Entkoppeleinrichtung für das Schlagelement befestigt werden.

Die US 6,010,513 beschreibt eine weitere Applikationsvorrichtung mit einem Schlagmechanismus, der über einen Betätigungsmechanismus gespannt und über eine separat bedienbare Entkoppeleinrichtung vom Betätigungsmechanismus entkoppelt wird.

Aus der US 5,258,010 ist ferner eine Applikationsvorrichtung bekannt, mit der klammerartige Implantate eingesetzt werden können. Hierzu weist die Vorrichtung einen Schlagmechanismus auf, der eine bewegbare Druckstange besitzt die bei Betätigung das Klammerimplantat aus der Vorrichtung treibt.

Die US 5,558,266 beschreibt ebenfalls eine Applikationsvorrichtung, mit der klammerartige Implantate eingesetzt werden können. Mittels eines speziellen Haltemechanismus werden zwei Haltebacken zueinander bewegt und fixieren das Gewebe, in das das selbsthaltende Implantat eingetrieben werden soll. Mit einem zweiten Mechanismus, dem Eintreibmechanismus, wird das Implantat aus einer Kartusche, die in einer der Backen aufgenommen ist, in das Gewebe eingetrieben.

Der Erfindung liegt die Aufgabe zugrunde, ein Implantatsystem anzugeben, welches ein selbsthaltendes Implantat mit einer vereinfachten Handhabbarkeit umfasst.

Diese Aufgabe wird erfindungsgemäß gelöst durch eine Vorrichtung gemäß Anspruch 1.

Das selbsthaltende Implantat besitzt ein Auflageelement mit einer Oberseite und einer dem Knochendeckel oder dem Knochenfragment zugewandten Unterseite. Auf der Unterseite des Auflageelements ist ein Fortsatz angeordnet, der mindestens eine, sich in Richtung auf den Knochendeckel erstreckende Spitze trägt. Die Spitze wird lateral in den Knochendeckel oder das Knochenfragment eingetrieben.

Die Eintreibvorrichtung umfasst eine Aufnahme für das selbsthaltende Implantat sowie einen an die Aufnahme koppelbaren Eintreibmechanismus zum Eintreiben des Implantats lateral in den Knochendeckel oder das Knochenfragment, der einen ersten Schlitten aufweist, der entlang eines ersten Führungsbolzens koaxial zur Aufnahme geführt wird und gegen eine Federkraft verschiebbar ausgebildet ist, um eine Schlagkraft auf die Aufnahme auszuüben und einen Betätigungsmechanismus für den Eintreibmechanismus. Der Eintreibmechanismus weist weiter einen zweiten Schlitten auf, der ebenfalls gegen eine Federkraft entlang eines zweiten Führungsbolzens verschiebbar ist, wobei der zweite Führungsbolzen sich parallel zu dem ersten Führungsbolzen erstreckt, eine Koppeleinrichtung zum Koppeln des Betätigungsmechanismus mit dem ersten Schlitten, wobei der Betätigungsmechanismus mit dem zweiten Schlitten gekoppelt ist, so dass die Koppeleinrichtung ein Koppeln des ersten Schlittens mit dem zweiten Schlitten ermöglicht, und wobei die Koppeleinrichtung dazu ausgebildet ist, den ersten Schlitten entgegen einer Federkraft vorzuspannen, und eine Entkoppelrichtung zum Entkoppeln des ersten Schlittens vor dem zweiten Schlitten, um den vorgespannten ersten Schlitten freizugeben.

Die Federkraft ermöglicht es hierbei, den ersten Schlitten in Eintreibrichtung vorzuspannen, wobei die Schlagkraft durch einen plötzlichen Abbau der Vorspannung aktivierbar ist. Diese wird durch ein Entkoppeln des ersten Schlittens aktiviert.

Der Führungsbolzen für den ersten Schlitten wird von einem koaxial zur Aufnahme angeordneten Bolzen gebildet. Die Aufnahme kann derart mit dem Bolzen oder einer anderen ortsfesten Komponente der Eintreibvorrichtung verbunden sein, dass die Aufnahme in axialer Richtung begrenzt beweglich ist. Die Koppeleinrichtung umfasst beispielsweise einen Mitnehmer für den ersten Schlitten.

Zudem ist eine korrespondierende Entkoppeleinrichtung vorhanden, welche zum Aktivieren der Schlagkraft ein Entkoppeln von ersten Schlitten und zweiten Schlitten gestattet.

Das erfindungsgemäße, selbsthaltende Implantat wird nicht mittels Aufschiebens am Knochendeckel befestigt, sondern mittels einer oder mehrerer in den Knochendeckel oder das Knochenfragment eingetriebener Spitzen. Das Eintreiben der mindestens einen Spitze des Implantats erfolgt vorzugsweise unter Verwendung einer erfindungsgemäßen Eintreibvorrichtung, welche zu diesem Zweck einen geeigneten Eintreibmechanismus umfassen kann. In der Praxis hat sich herausgestellt, dass ein Eintreiben des selbsthaltenden Implantats, insbesondere bei Verwendung einer erfindungsgemäßen Eintreibvorrichtung, aus Gesichtspunkten der Handhabung besonders vorteilhaft ist. Die aus dem Eintreiben der Spitze des Implantats in z.B. den spongiösen oder kortikalen Bereich des Knochendeckels oder des Knochenfragments resultierende Verbindung zwischen dem Implantat und dem Knochendeckel oder dem Knochenfragment ist äußerst stabil und zuverlässig.

Die mindestens eine Spitze des selbsthaltenden Implantats ist bezüglich des Auflageelements derart angeordnet, dass sie zuverlässig in den Knochendeckel oder das Knochenfragment eingetrieben werden kann und eine gute Verankerung des Implantats gewährleistet. Zu diesem Zweck erstreckt sich die Spitze vorzugsweise im wesentlichen parallel zum Auflageelement. Die Spitze kann jedoch bezüglich des Auflageelements auch geneigt sein, sofern eine ausreichende Eintreibbarkeit gewährleistet ist. Der Übergang zwischen dem Fortsatz und der am Fortsatz angeordneten Spitze kann kontinuierlich sein, so dass keine definierte Grenze zwischen Fortsatz und Spitze erkennbar ist. Gemäss einer bevorzugten Ausführungsform steht die Spitze jedoch unter einem Winkel von beispielsweise 90° vom Fortsatz ab.

Der Fortsatz schließt mit dem Auflageelement zweckmäßigerweise einen Winkel zwischen 45° und 135° ein. Bevorzugt ist ein Fortsatz, welcher sich im wesentlichen rechtwinkelig zum Auflageelement erstreckt. In diesem Fall kann der Fortsatz als Anschlag fungieren, welcher ein definiertes Eintreiben der Spitze ermöglicht. Mit anderen Worten: sobald der Fortsatz in Anlage an laterale Bereiche des Knochendeckels oder des Knochenfragments gelangt, verhindert er ein weiteres Eintreiben der Spitze. Außerdem gestattet eine Analyse der Lage des Fortsatzes relativ zu einem lateralen Bereich des Knochendeckels oder des Knochenfragments eine visuelle Beurteilung, ob die Spitze des Implantats ausreichend tief eingetrieben wurde.

Gemäss einer bevorzugten Ausführungsform ist der Fortsatz derart relativ zum Auflageelement angeordnet, dass das Auflageelement und der Fortsatz eine im Querschnitt im wesentlichen T-förmige Struktur bilden. Das Auflageelement entspricht dabei dem Querbalken des T und der Fortsatz dessen Längsbalken. Der Fortsatz unterteilt das Auflageelement somit in zwei sich in entgegengesetzte Richtungen erstreckende Auflagearme. Ein erster der beiden Auflagearme verläuft in Richtung auf den Knochendeckel und ein zweiter der beiden Auflagearme in Richtung auf den Schädelknochen. Eine derartige Ausgestaltung des Implantats ist vorteilhaft, da der sich in Richtung auf den Knochendeckel erstreckende und auf der Knochendeckeloberseite aufliegende Auflagearm zusammen mit der in den spongiösen Bereich des Knochendeckels eingetriebenen Spitze eine besonders zuverlässige Befestigung des Implantats am Knochendeckel ermöglicht. Außerdem kann der mit dem Knochendeckel zusammenwirkende Auflagearm als Führung während des Positionierens sowie des Eintreibens des Implantats verwendet werden. Anstatt T-förmig könnte das Implantat jedoch auch z.B. Z-förmig ausgestaltet werden, wobei der obere Querbalken des Z als Auflageelement auf dem Schädelknochen fungiert und der untere Querbalken die Spitze bildet.

Das Auflageelement besitzt vorzugsweise eine flächige, z.B. eine streifenförmige Gestalt. Die Unterseite des Auflageelements kann zumindest in Bereichen konkav oder sphärisch gekrümmt sein, um eine gute Anpassung des Implantats an die Schädelrundung zu gewährleisten. Zweckmäßigerweise ist das Auflageelement des Implantats zumindest bereichsweise biegsam ausgestaltet, um eine individuelle Anpassung des Implantats an die Schädelrundung zu ermöglichen.

Die einzutreibende Spitze des Implantats ist z.B. an einem dem Auflageelement abgewandten Ende des Fortsatzes angeordnet. Die Spitze kann unterschiedlich ausgestaltet sein. So ist es beispielsweise möglich, eine Spitze in Gestalt eines oder mehrerer Dorne oder einer Sägezahnstruktur vorzusehen. Vorzugsweise besitzt die Spitze jedoch eine flächige, annähernd dreieckige Gestalt. Um das Eintreiben der Spitze zu erleichtern, kann die Spitze mit geschärften Kanten versehen werden. Die Länge der Spitze ist in weiten Bereichen frei wählbar, sofern eine ausreichend zuverlässige Verankerung des Implantats gewährleistet ist.

An seinem der Spitze entgegengesetzten Ende kann das Auflageelement ein Schraubenloch besitzen, um ein Festlegen des mit einem oder mehreren Implantaten versehenen Knochendeckels oder Knochenfragments mittels Schrauben am Schädelknochen zu ermöglichen. Bei einem Auflageelement mit zwei oder mehr Auflagearmen ist das Schraubenloch folglich in demjenigen Auflagearm ausgebildet, welcher mit dem Schädelknochen zusammenwirkt. Vorzugsweise besitzt das Auflageelement im Bereichs des Schraubenlochs eine lokal höhere Stärke. Dabei kann die Stärke des Auflageelements im Bereich des Schraubenlochs derart gewählt sein, dass der Schraubenkopf bereichsweise oder vollständig im Schraubenloch versenkbar ist. Dies ist aus kosmetischen Gründen vorteilhaft. In Bereichen des Auflageelements außerhalb des Schraubenlochs kann die Stärke des Auflageelements hingegen deutlich geringer gewählt werden. Das Innere des Schraubenlochs ist zweckmäßigerweise sphärisch gekrümmt, um das Versenken von Knochenschraubenköpfe mit einer korrespondierenden sphärischen Krümmung zu ermöglichen.

Das erfindungsgemäße Implantat kann auf unterschiedliche Weise in den Knochendeckel oder das Knochenfragment eingetrieben werden. Mittels der erfindungsgemäßen, einen Eintreibmechanismus umfassenden Eintreibvorrichtung ist dies auf besonders einfache Weise möglich. Der Eintreibmechanismus kann derart ausgestaltet sein, dass er das Ausüben der Schlagkraft auf die für das Implantat vorgesehene Aufnahme gestattet. Weiterhin kann er hierbei mit einer mit der Aufnahme kraftübertragend gekoppelten Krafteinleitungsfläche derart gekoppelt sein, dass die Schlagkraft vom Eintreibmechanismus in die Krafteinleitungsfläche, von dieser in die Aufnahme und von der Aufnahme in das Implantat übertragen wird.

Die Eintreibvorrichtung umfasst einen Betätigungsmechanismus für den Eintreibmechanismus. Dabei wird die zum Verschieben des ersten Schlittens benötigte Kraft vorzugsweise mittels des Betätigungsmechanismus aufgebracht. Der Betätigungsmechanismus kann zu diesem Zweck mit Hilfe eines Elektromotors oder eines oder mehrerer Finger betätigbar sein.

Weiterhin ist vorgesehen, dass die Koppeleinrichtung zwischen dem Betätigungsmechanismus und dem ersten Schlitten angeordnet ist, wobei die Koppeleinrichtung vorzugsweise vorgespannt ist, um den Betätigungsmechanismus mit dem ersten Schlitten zu koppeln.

Gemäss einer besonders bevorzugten Ausführungsform weist die erfindungsgemäße Eintreibvorrichtung einen pistolenartigen Aufbau auf. Eine derartige Formgebung gestattet ein besonders ergonomisches und sicheres Eintreiben der Implantate. Andere Formgebungen der erfindungsgemäßen Eintreibvorrichtung sind jedoch ebenfalls denkbar. So kann die Eintreibvorrichtung beispielsweise eine im wesentlichen zylindrische Gestalt besitzen.

Eine Eintreibvorrichtung mit pistolenartigem Aufbau besitzt typischerweise einen Pistolenkörper und einen Pistolenlauf. Der Pistolenkörper kann einen Pistolenhandgriff sowie ein mit dem Handgriff verbundenes Gehäuse zur Aufnahme des Eintreibmechanismus besitzen. Der Betätigungsmechanismus für den Eintreibmechanismus kann als fingerbetätigbarer Pistolenabzug ausgebildet sein. Vorzugsweise ist der Pistolenabzug mit dem gegen eine Federkraft verschiebbaren, zweiten Schlitten gekoppelt. Die vorstehend beschriebene Koppeleinrichtung kann in diesem Fall funktionell zwischen dem ersten und dem zweiten Schlitten angeordnet sein.

Bei einer pistolenartigen Ausgestaltung der Eintreibvorrichtung ist es möglich, die Aufnahme für das Implantat nach Art eines Pistolenlaufs bezüglich des Pistolenkörpers anzuordnen. Eine derartige Anordnung der Aufnahme gestattet ein besonders einfaches Positionieren des einzutreibenden Implantats bezüglich des Knochendeckels oder des Knochenfragments.

Die Aufnahme kann mit einem Selbsthaltemechanismus für das Implantat versehen sein, so dass das Implantat unmittelbar aus einem hierfür vorgesehenen Magazin mittels der Eintreibvorrichtung entnommen werden kann. Es kann daher während des gesamten Befestigungsvorgangs des Implantats darauf verzichtet werden, das Implantat mit den Fingern zu berühren. Dies ist aus ergonomischen Gesichtspunkten wünschenswert.

Nachfolgend wird ein Ausführungsbeispiel des Implantats und ein Ausführungsbeispiel der Vorrichtung anhand mehrerer Figuren näher erläutert. Es zeigt:
Fig. 1 eine Seitenansicht eines Ausführungsbeispiels des erfindungsgemäßen Implantats;
Fig. 2 eine Aufsicht auf die der Knochenplatte zugewandten Unterseite des Implantats gemäss Fig. 1;
Fig. 3 eine Schnittansicht durch das Auflageelement des Implantats gemäss den Fig. 1 und 2 im Bereich eines Schraubenlochs;
Fig. 4 eine Seitenansicht eines ersten Ausführungsbeispiels einer erfindungsgemäßen Eintreibvorrichtung;
Fig. 5 einen Längsschnitt durch die Eintreibvorrichtung gemäss Fig. 4;
Fig. 6 einen Querschnitt durch die Eintreibvorrichtung gemäss Fig. 4 entlang der Linie VI-VI gemäss Fig. 5;
Fig. 7a und 7b die Aufnahme für das Implantat in einer Aufsicht und einer Schnittansicht;
Fig. 8 ein zweites Ausführungsbeispiel einer erfindungsgemäßen Eintreibvorrichtung in einer Aufsicht; und
Fig. 9 eine Schrägansicht des eingetriebenen Implantats gemäss Fig. 1 nach dessen Befestigung im Schädelknochen.

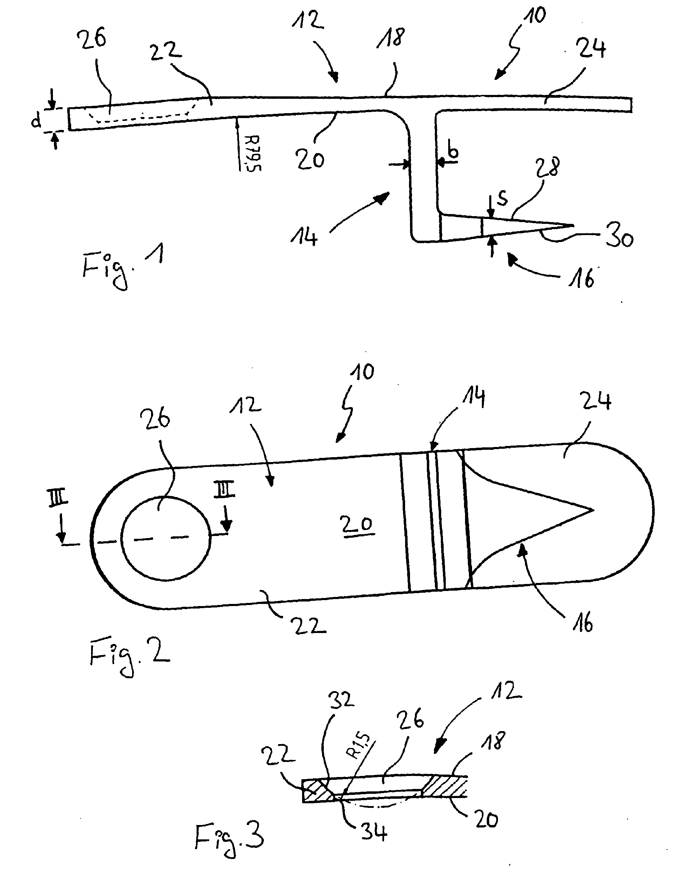

In Fig. 1 ist ein Ausführungsbeispiel eines selbsthaltenden Implantats 10 in einer Seitenansicht dargestellt. Das Implantat 10 umfasst ein Auflageelement 12, einen mit dem Auflageelement 12 verbundenen Fortsatz 14 und eine mit dem Fortsatz 14 verbundene Spitze 16.

Das Auflageelement 12 besitzt eine Oberseite 18 und eine Unterseite 20. Die Unterseite 20 ist nach der Implantation einem in Fig. 1 nicht dargestellten Knochendeckel oder Knochenfragment sowie einem in Fig. 1 ebenfalls nicht dargestellten Schädelknochen zugewandt. Um eine möglichst spielfreie Auflage der Unterseite 20 zu gewährleisten, ist diese sphärisch gekrümmt. Der Krümmungsradius R beträgt 79,5 mm.

Das Auflageelement 12 setzt sich aus zwei Auflagearmen 22, 24 zusammen. Der in Fig. 1 linke Auflagearm 22 liegt im implantierten Zustand des Implantats 12 auf dem Schädelknochen auf und der in Fig. 1 rechte Auflagearm 24 auf z.B. dem Knochendeckel. Das Längenverhältnis zwischen dem linken Auflagearm 22 und dem rechten Auflagearm 24 lässt sich mittels der Position des Fortsatzes 14 relativ zu den beiden Auflagearmen 22, 24 variieren. In der Darstellung gemäss Fig. 1 besitzt der linke Auflagearm 22 eine etwas größere Länge als der rechte Auflagearm 24.

Der linke Arm 22 ist an seinem der Spitze 16 abgewandten Ende mit einem Schraubenloch 26 versehen. Im Bereich des Schraubenlochs 26 besitzt das Auflageelement 12 seine maximale Stärke d von ungefähr 0,5 mm. Diese Stärke wurde gewählt, um ein kosmetisch vorteilhaftes Versenken eines Knochenschraubenkopfes im Auflageelement 12 zu ermöglichen. In vom Schraubenloch 26 beabstandeten Bereichen besitzt das Auflageelement 12 eine geringere Stärke von typischerweise 0,3 mm. Diese geringere Stärke ist derart gewählt, dass das Auflageelement in Bereichen außerhalb des Schraubenlochs geringfügig gebogen werden kann. Mittels Biegens des Auflageelements 12 kann eine individuelle Anpassung der Unterseite 20 an die Krümmung des Knochendeckels oder Schädelknochens erfolgen.

Der Fortsatz 14 des Implantats 10 ist an der Unterseite 20 des Auflageelements 12 befestigt und erstreckt sich im wesentlichen rechtwinkelig zum Auflageelement 12. Das Auflageelement 12 und der Fortsatz 14 bilden zusammen eine T-förmige Struktur. Dabei entspricht das Auflageelement 12 dem Querbalken der T-förmigen Struktur und der Fortsatz 14 dem Längsbalken.

Die Breite b des Fortsatzes 14 sollte gering sein, damit der Knochenspalt zwischen dem Knochendeckel oder dem Knochenfragment und dem Schädelknochen klein gehalten werden kann. Zweckmäßig sind Breiten b von weniger als einem Millimeter, beispielsweise von 0,6 mm. Mittels des Implantats 10 kann der Knochendeckel oder das Knochenfragment derart im Schädelknochen positioniert werden, dass der Knochenspalt entlang der Kontur des Knochendeckels oder des Knochenfragments konstant ist. Um ein schnelleres Anwachsen des Knochendeckels oder des Knochenfragments sicherzustellen, kann der Knochendeckel oder das Knochenfragment jedoch auch mittels des Implantats 10 in bereichsweisem Kontakt mit dem Schädelknochen festgelegt werden.

Wenn das Auflageelement 12 oder der Fortsatz 14 mit der Eintreibkraft, welche in Fig. 1 nach rechts wirkt, beaufschlagt wird, wird die Eintreibkraft vom Fortsatz 16 auf die Spitze 16 zu übertragen. Die Spitze 16 ist an demjenigen Ende des Fortsatzes 14 ausgebildet, welches dem Auflageelement 12 gegenüberliegt. In Fig. 1 ist deutlich zu erkennen, dass sich die Spitze senkrecht zum Fortsatz 14 und im wesentlichen parallel zum Auflageelement 12 erstreckt. Die Stärke s der Spitze 16 nimmt mit zunehmendem Abstand vom Fortsatz 14 allmählich ab. Die Kanten 28, 30 der Spitze 16 sind geschärft, um das Eintreiben der Spitze in den Knochendeckel oder das Knochenfragment zu erleichtern.

In Fig. 2 ist das Implantat 10 gemäss Fig. 1 in einer Aufsicht auf die Unterseite 20 des Auflageelements 12 dargestellt. Wie Fig. 2 entnommen werden kann, besitzt das Auflageelement 12 eine im wesentlichen streifenförmige Gestalt mit abgerundeten Enden. Die Spitze 16 ist dreieckig ausgestaltet und kann daher mit vergleichsweise geringer Eintreibkraft eingebracht werden. Alternative Ausgestaltungen der Spitze 16 sind ebenfalls denkbar. So könnte die Spitze 16 beispielsweise zusätzlich mit widerhakenartigen Strukturen versehen sein, um die Verankerung der Spitze 16 im spongiösen Bereich zu verbessern.

Fig. 3 zeigt einen Schnitt durch das in Fig. 2 dargestellte Implantat im Bereich des Schraubenlochs 26. Das Innere des Schraubenlochs 26 besitzt eine sphärisch gekrümmten Bereich 32, welcher in Richtung auf die Unterseite 20 in einen kurzen, zylindrischen Bereich 34 mündet. Der sphärisch gekrümmte Bereich 32 ermöglicht das Versenken von korrespondierend geformten Knochenschraubenköpfen im Auflageelement 12.

Das Implantat 10 gemäss den Fig. 1 bis 3 ist selbsthaltend, da es ohne Befestigungselemente wie Schrauben, Nägel, usw. an z.B. einem Knochendeckel befestigbar ist. Im Gegensatz zu den klammerartigen Implantaten des Stands der Technik ist die Haltekraft des Implantats 10 im wesentlichen unabhängig von der Stärke des Knochendeckels. So kann auch bei geringen Knochendeckelstärken eine feste Verankerung des erfindungsgemäßen Implantats gewährleistet werden. Das Implantat 10 wird lateral in den Knochendeckel oder in das Knochenfragment eingetrieben. Die Verankerung des Implantats 10 erfordert daher keine auf der Unterseite des Knochendeckels oder des Knochenfragments aufliegende Elemente, so dass eine Verletzung der Hirnhautlappen durch das Implantat 10 ausgeschlossen ist.

Die Länge des Fortsatzes 14, d.h. der Abstand zwischen der Spitze 30 und dem Auflageelement 12, ist typischerweise derart gewählt, dass das Implantat 10 bei Knochendeckelstärken bis 3,5 mm sicher verankerbar ist. Durch Bereitstellen von Implantaten 10 mit kürzeren Fortsätzen 14 können auch Knochendeckel mit einer Stärke von weniger als 3,5 mm festgelegt werden. Das Implantat 10 ist aus biokompatiblem TiAl6V4 hergestellt. Anstatt aus einem Metall kann das Implantat 10 jedoch auch ganz oder bereichsweise aus einem resorbierbaren Material bestehen.

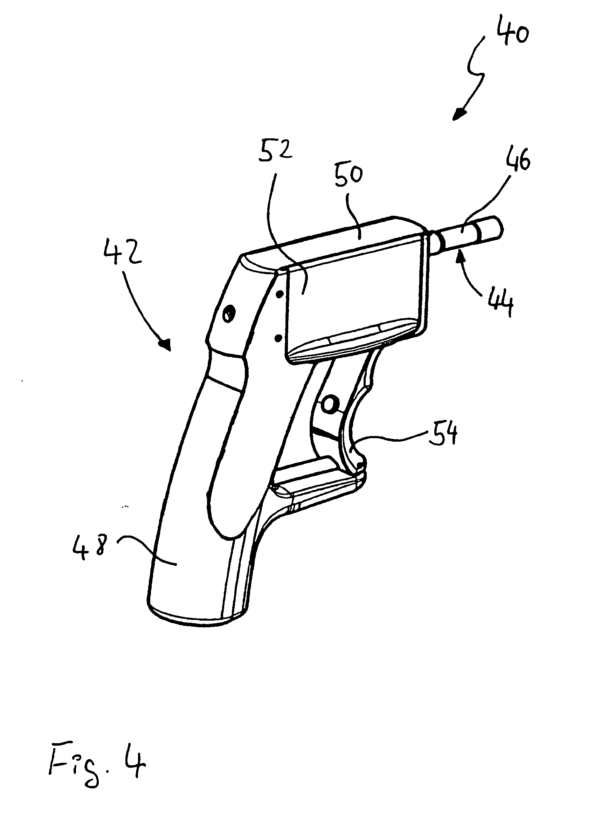

In Fig. 4 ist ein erstes Ausführungsbeispiel einer erfindungsgemäßen Eintreibvorrichtung 40 für das Implantat 10 gemäss den Fig. 1 bis 3 dargestellt. Die Eintreibvorrichtung 40 besitzt die Form einer Pistole mit einem Pistolenkörper 42 und einem Pistolenlauf 44. Der Pistolenlauf 44 wird von einer Aufnahme 46 für das einzutreibende Implantat gebildet. Der Pistolenkörper 42 setzt sich aus einem Handgriff 48 und einem Gehäuse 50 zusammen. Innerhalb des Gehäuses 50 ist der in Fig. 4 nicht dargestellte, mit der Aufnahme 46 zusammenwirkende Eintreibmechanismus angeordnet. Der Eintreibmechanismus gestattet das Einleiten einer Eintreibkraft in die Aufnahme 46.

Das Gehäuse 50 umfasst zwei seitlich angeordnete und vollständig abnehmbare Gehäusedeckel. In der Ansicht gemäss Fig. 4 ist ein einziger Gehäusedeckel 52 zu erkennen. Die abnehmbaren Gehäusedeckel gestatten eine einfache Reinigung und Schmierung des Eintreibmechanismus im Anschluss an die Operation.

Für die Betätigung des Eintreibmechanismus ist ein Betätigungsmechanismus in Gestalt eines Pistolenabzugs 54 vorgesehen. Der Pistolenabzug 54 besitzt eine ergonomische Form, welche an eine Aktivierung mittels Zeigefinger und Mittelfinger oder mittels Mittelfinger und Ringfinger angepasst ist.

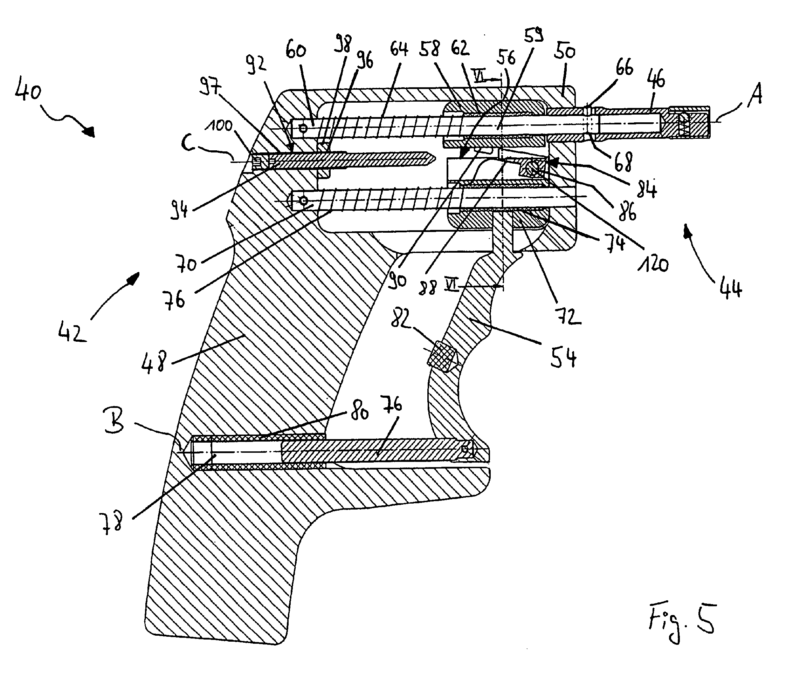

Fig. 5 zeigt einen Längsschnitt durch die Eintreibvorrichtung 40 gemäss Fig. 4 und insbesondere den Eintreibmechanismus 56 der Eintreibvorrichtung 40.

Der Eintreibmechanismus 56 ist als Schlagmechanismus ausgestaltet und umfasst ein Schlagelement in Gestalt eines oberen Schlittens 58. Der obere Schlitten 58 besitzt eine zentrale Durchgangsöffnung 59, durch welche sich ein am Gehäuse 50 verankerter, ortsfester, oberer Führungsbolzen 60 erstreckt. Der obere Schlitten 58 ist mittels eines Kugellagers 62 entlang des oberen Führungsbolzens 60 verschiebbar geführt.

Der obere Führungsbolzen 60 ist radial außen von einer Schraubenfeder 64 umgeben, welche in der in Fig. 5 dargestellten Ausgangsstellung des Eintreibmechanismus 56 mit ihrem einen Ende am Gehäuse 50 und mit ihrem anderen Ende am oberen Schlitten 58 anliegt. Bei einer Bewegung des Schlittens entlang des oberen Führungsbolzens 60 in Fig. 5 nach links wird die Schraubenfeder 64 komprimiert. Gleichzeitig wird der obere Schlitten 58 in Fig. 5 nach rechts, d.h. in Eintreibrichtung, vorgespannt.

Der obere Führungsbolzen 60 ragt pistolenlaufartig teilweise aus dem Gehäuse 50 heraus. An seinem aus dem Gehäuse 50 herausragenden Ende ist der obere Führungsbolzen 60 mit der dieses Ende radial außen umgebenden Aufnahme 46 für das Implantat gekoppelt. Die Kopplung des oberen Führungsbolzens 60 mit der Aufnahme 46 geschieht mittels eines starr mit dem oberen Führungsbolzen 60 verbundenen Querbolzens 66. Der Querbolzen 66 ragt durch eine Öffnung 68 der Aufnahme 46. Die Öffnung 68 ist ein Längsloch mit einer ovalen Gestalt und ermöglicht daher eine begrenzte Bewegung der Aufnahme 46 relativ zum oberen Führungsbolzen 60 entlang der Achse A. Die relative Beweglichkeit der Aufnahmehülse ist erforderlich, um eine in die Aufnahmehülse 46 eingeleitet Schlagkraft auf das von der Aufnahme 46 aufgenommene, in Fig. 5 nicht dargestellte Implantat weiterzuleiten. Die Schlagkraft wird in die Aufnahme 46 mittels des koaxial zur Aufnahme 46 angeordneten, oberen Schlittens 58 eingeleitet.

Innerhalb des Gehäuses 50 ist etwas unterhalb des oberen Führungsbolzens 60 ein mittlerer Führungsbolzen 70 angeordnet. Der mittlere Führungsbolzen 70 ist ortsfest im Gehäuse 50 befestigt und erstreckt sich parallel zum oberen Führungsbolzen 60. Ein unterer Schlitten 72 ist mittels eines Kugellagers 74 entlang des mittleren Führungsbolzens 70, welcher den unteren Schlitten 72 durchgreift, geführt beweglich. Radial außen ist der mittlere Führungsbolzen 70 von einer Schraubenfeder 76 umgeben, welche sich einerseits am Gehäuse 50 und andererseits am unteren Schlitten 72 abstützt. Bei einer Verschiebung des unteren Schlittens 72 entlang des mittleren Führungsbolzens 70 in Fig. 5 nach links wird die Schraubenfeder 76 komprimiert. Gleichzeitig wird der untere Schlitten 72 in Fig. 5 nach rechts vorgespannt.

Der untere Schlitten 72 ist starr mit dem Pistolenabzug 54 gekoppelt. Bei einer Betätigung des Pistolenabzugs 54, d.h. bei einer Verschiebung des Pistolenabzugs 54 in Fig. 5 nach links, wird der untere Schlitten 72 daher ebenfalls nach links verschoben. In den Pistolenabzug 54 ist ein Kunststoffpfropfen 82 eingepresst. Der Kunststoffpfropfen 82 gelangt bei eine Betätigung des Pistolenabzugs 54 in Anlage an den Pistolengriff 48 und begrenzt damit die Beweglichkeit des Pistolenabzugs 54.

Der Pistolenabzug 54 ist an seinem in Fig. 5 unteren Ende mit einem relativ zum Gehäuse 50 beweglichen, unteren Führungsbolzen 76 verbunden. Der untere Führungsbolzen 76 ist in einer im Pistolengriff 48 ausgebildeten Sacklochbohrung 78 entlang einer Achse B verschiebbar geführt. Zur Reduzierung des Reibungswiderstands ist in die Sacklochbohrung 78 eine Kunststoffhülse 80 eingepresst, welche die Sacklochbohrung 78 radial innen auskleidet.

Innerhalb des Gehäuses 50 ist eine Koppeleinrichtung 84 vorgesehen. Die Koppeleinrichtung 84 gestattet es, den Betätigungsmechanismus, d.h. den Pistolenabzug 54, mit dem Schlagelement in Gestalt des oberen Schlittens 58 zu koppeln. Die Koppeleinrichtung 84 umfasst einen in der Zeichenebene schwenkbaren Mitnehmer 86, eine Blattfeder 88 sowie ein Koppelelement 90.

Der Mitnehmer 86 ist schwenkbar am unteren Schlitten 72 befestigt und wird von der ebenfalls am unteren Schlitten 72 befestigten Blattfeder 88 in Richtung auf den oberen Schlitten 58 vorgespannt. Am oberen Schlitten 58 ist das mit dem Mitnehmer 86 zusammenwirkende Koppelelement 90 angeordnet.

Innerhalb des Gehäuses 50 ist außerdem eine Entkoppeleinrichtung 92 untergebracht, welche mit der Koppeleinrichtung 84 zusammenwirken und die Kopplung zwischen Pistolenabzug 54 und dem oberen Schlitten 58 aufheben kann. Die Entkoppeleinrichtung 92 umfasst einen in das Gehäuse 50 ragenden Dorn 94. Um die Entkoppelschwelle verstellbar auszugestalten, ist der Dorn 94 entlang seiner Längsachse C beweglich. Zu diesem Zweck besitzt der Dorn 94 radial außen ein Außengewinde 96, welches mit einem komplementären Innengewinde 97 im Pistolenkörper 42 zusammenwirkt. Eine Kontermutter 98 gestattet die Arretierung des Dorns 94 in einer gewünschten, axialen Position. Der Dorn 94 ist an seinem der Dornspitze abgewandten Ende mit einem Innenmehrkant versehen, um zur Einstellung der Entkoppelschwelle den Angriff eines Werkzeugs am Dorn 94 zu ermöglichen.

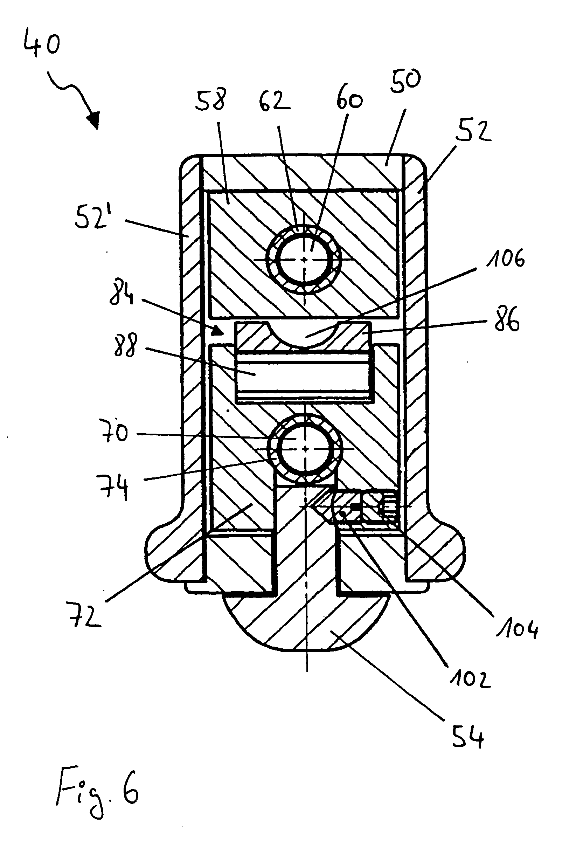

In Fig. 6 ist ein Querschnitt durch die in Fig. 4 abgebildete Eintreibvorrichtung 40 entlang der Schnittlinie IV-IV gemäss Fig. 5 dargestellt. Wie Fig. 6 entnommen werden kann, ist das Gehäuse 50 von zwei entfernbaren Gehäusedeckeln 52, 52' seitlich verschlossen. Innerhalb des Gehäuses 50 ist der Eintreibmechanismus mit dem entlang des oberen Führungsbolzens 60 geführten oberen Schlitten 58 und dem entlang des mittleren Führungsbolzens 70 geführten unteren Schlitten 72 angeordnet. Der untere Schlitten 72 ist mittels einer Schraube 102 mit dem Pistolenhandgriff 54 verbunden. Die Schraube 102 besitzt einen Innenmehrkant 104.

In Fig. 6 ist deutlich zu erkennen, dass der mit der Blattfeder 88 zusammenwirkende Mitnehmer 86 der Koppeleinrichtung 84 eine U-förmige Nut 106 besitzt. Mittels dieser Nut 106 wirkt die Koppeleinrichtung 84 mit dem Dorn 94 der Entkoppeleinrichtung zusammen. Die Nut 106 ist bezüglich der Zeichenebene derart angeordnet, dass der Mitnehmer 86 von der Spitze des Dorns 94 entgegen der Federkraft der Blattfeder 88 in Fig. 6 nach unten gedrückt werden kann.

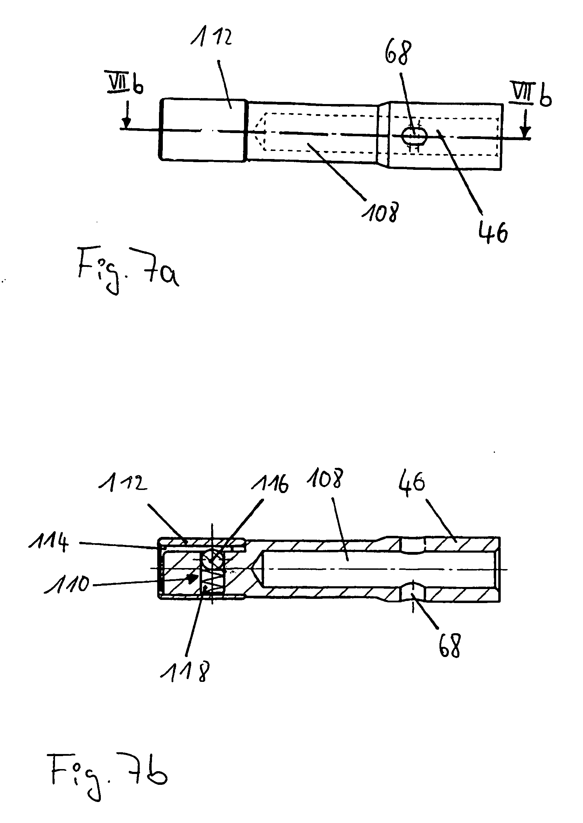

In den Fig. 7a und 7b ist die Aufnahme 46 der in Fig. 4 dargestellten Eintreibvorrichtung 40 abgebildet. Zu erkennen ist die ovale Öffnung 68, in welcher der in Fig. 5 dargestellte Querbolzen 66 beweglich geführt ist. Die Aufnahme 46 ist bereichsweise hülsenförmig ausgestaltet und besitzt eine Sacklochbohrung 108 zur Aufnahme eines Endbereichs des in Fig. 5 dargestellten oberen Führungsbolzens 60. An seinem der Sacklochbohrung 108 gegenüberliegenden Ende besitzt die Aufnahme 46 eine Hülse 112, welche den zylindrischen Grundkörper der Aufnahme 46 radial außen umgibt. Zwischen dem zylindrischen Grundkörper der Aufnahme 46 und der Hülse 112 ist ein ebener Schlitz 114 ausgebildet, welcher zu Aufnahme des Implantats dient. Der Schlitz 114 ist in der Schnittansicht gemäss Fig. 7b zu erkennen.

Im Bereich des Schlitzes 114 ist ein Selbsthaltemechanismus 110 angeordnet. Der Selbsthaltemechanismus 110 umfasst eine Kugel 116 sowie eine die Kugel 116 in Richtung auf den Schlitz 114 vorspannende Feder 118. Die Kugel 116 wirkt mit dem in Fig. 2 dargestellten Schraubenloch 26 des Implantats 10 zusammen.

Nachfolgend wird die Funktionsweise der vorstehend unter Bezugnahme auf die Fig. 4 bis 7b beschriebenen Eintreibvorrichtung 40 näher erläutert.

In einem ersten Schritt wird das in den Fig. 1 bis 3 dargestellt Implantat 10 mit seinem das Schraubenloch 26 aufweisenden linken Auflagearm 22 in den Schlitz 114 der Aufnahme 46 eingeführt. Sobald das Schraubenloch 26 im Bereich der in Richtung auf den Schlitz 114 vorgespannten Kugel 116 liegt, greift die Kugel 116 in das Schraubenloch 26 ein und der Selbsthaltemechanismus 110 ist aktiviert. Da die Kugel 116 unter Vorspannung in das Schraubenloch des Implantats eingreift, kann das Implantat nicht mehr versehentlich aus dem Schlitz 114 gleiten.

Vorzugsweise wird das Implantat unmittelbar mittels der Eintreibvorrichtung 40 aus einem Implantatcontainer entnommen. Das Implantat muss folglich nicht unter Zuhilfenahme der Finger in den Schlitz 114 eingeführt werden. Die Handhabung des erfindungsgemäßen Implantatsystems aus Implantat und Eintreibvorrichtung 40 wird dadurch wesentlich verbessert.

Nachdem das Implantat in den Schlitz 114 eingeführt und dort mittels des Selbsthaltemechanismus 110 gegen ein Herausfallen gesichert ist, wird das gesicherte Implantat mittels der Eintreibvorrichtung 40 im Bereich des festzulegenden Knochendeckels oder Knochenfragments positioniert. Die Positionierung erfolgt derart, dass der in Fig. 1 dargestellte, rechte Auflagearm 24 des Implantats 10 auf der Oberfläche des Knochendeckels oder Knochenfragments aufliegt und die Spitze lateral den spongiösen oder kortikalen Bereich kontaktiert.

Im Anschluss daran wird der Pistolenabzug 54 mittels zweier Finger in Fig. 5 nach links bewegt. Von dieser Bewegung des Pistolenabzugs 54 wird auch der starr mit dem Pistolenabzug 54 gekoppelte untere Schlitten 52 erfasst. Dieser bewegt sich folglich entgegen der Federkraft der Schraubenfeder 76 in Fig. 5 nach links. Aufgrund der Verschiebung des unteren Schlittens 72 in Fig. 5 nach links gelangt der Mitnehmer 86 in Kontakt mit dem Koppelelement 90 des oberen Schlittens 58. Sobald der Mitnehmer 84 am Koppelelement 90 anliegt, wird der obere Schlitten 58 vom Mitnehmer 86 mitgenommen und entgegen der Federkraft der Schraubenfeder 64 ebenfalls in Fig. 5 nach links verschoben.

Die Kopplung des unteren Schlittens 72 mit dem oberen Schlitten 58 wird vom Benutzer der Eintreibvorrichtung 40 dadurch zur Kenntnis genommen, dass die für die Betätigung des Pistolenabzugs 54 erforderliche Kraft ansteigt. Dieser Kraftanstieg ist darauf zurückzuführen, dass infolge der Kopplung von unterem Schlitten 72 und oberem Schlitten 58 nicht mehr nur die untere Schraubenfeder 76, sondern nunmehr zusätzlich die obere Schraubenfeder 84 komprimiert werden muss.

Nach einer gewissen Verschiebung der beiden Schlitten 72, 58 gelangt der Dorn 94 in Kontakt mit der in Fig. 6 dargestellten Nut 108 des Mitnehmers 86. Bei einer weiteren Verschiebung der beiden Schlitten 58, 72 in Fig. 5 nach links wird der Mitnehmer 86 nun von der Schrägfläche der Spitze des Dorns 94 um dessen in Fig. 5 dargestellte Schwenkachse 120 gegen die Federkraft der Blattfeder 88 nach unten geschwenkt. Bei einem Grenzschwenkwinkel wird schließlich die Kopplung zwischen dem Mitnehmer 86 und dem Koppelelement 90 des oberen Schlittens 58 abrupt aufgehoben. Infolge der Entkopplung von unterem Schlitten 72 und oberem Schlitten 58 wird der von der Schraubenfeder 64 in Fig. 5 nach rechts vorgespannte obere Schlitten 58 in Fig. 5 entlang des oberen Führungskolbens 60 nach rechts beschleunigt. Der beschleunigte obere Schlitten 58 gelangt schlagartig in Anlage mit der dem oberen Schlitten 58 zugewandten Stirnseite des entlang der Achse A geringfügig beweglichen Aufnehmers 46 und übt einen Schlag auf diesen aus. Dieser Schlag überträgt sich auf das im Schlitz 114 angeordnete Implantat 10, dessen Spitze 16 daraufhin lateral in den Knochendeckel oder das Knochenfragment eingetrieben wird. Während des Eintreibens wirkt der auf dem Knochendeckel oder Knochenfragment aufliegende Auflagearm 24 des Implantats 10 als Führung.

Sobald der obere Schlitten 58 vom unteren Schlitten 72 entkoppelt ist, kann der Pistolenabzug 54 wieder frei gegeben werden. Nach Freigabe des Pistolenabzugs 54 dehnt sich die komprimierte Schraubenfeder 76 wieder aus und der Pistolenabzug 54 wird von der Schraubenfeder 76 in die in Fig. 5 dargestellte Ausgangsposition zurückbewegt. Im Anschluss daran kann der vorstehend beschrieben Eintreibvorgang wiederholt werden, um erforderlichenfalls die Spitze 16 des Implantats 10 noch tiefer einzutreiben. Sobald der Fortsatz 14 lateral am Knochendeckel oder Knochenfragment anliegt, ist der Eintreibvorgang beendet. Der Fortsatz 14 fungiert dann als mechanischer Anschlag, der verhindert, dass die Spitze 16 zu tief in den Knochendeckel eindringt.

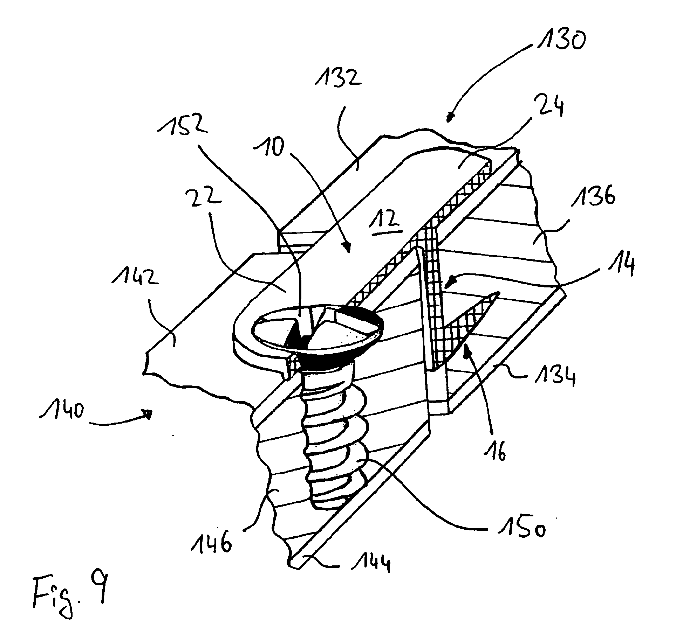

Üblicherweise müssen drei bis vier Implantate 10 gemäss den Fig. 1 bis 3 in einem Knochendeckel positioniert werden, um diesen zuverlässig im Schädelknochen festlegen zu können. Nachdem die erforderliche Anzahl von Implantaten in den Knochendeckel eingetrieben wurde, wird der Knochendeckel in der korrespondierenden Öffnung des Schädelknochens positioniert und mit je einer Knochenschraube pro Implantat am Schädelknochen befestigt. Dies ist in Fig. 9, welche weiter unten ausführlicher beschrieben wird, näher dargestellt.

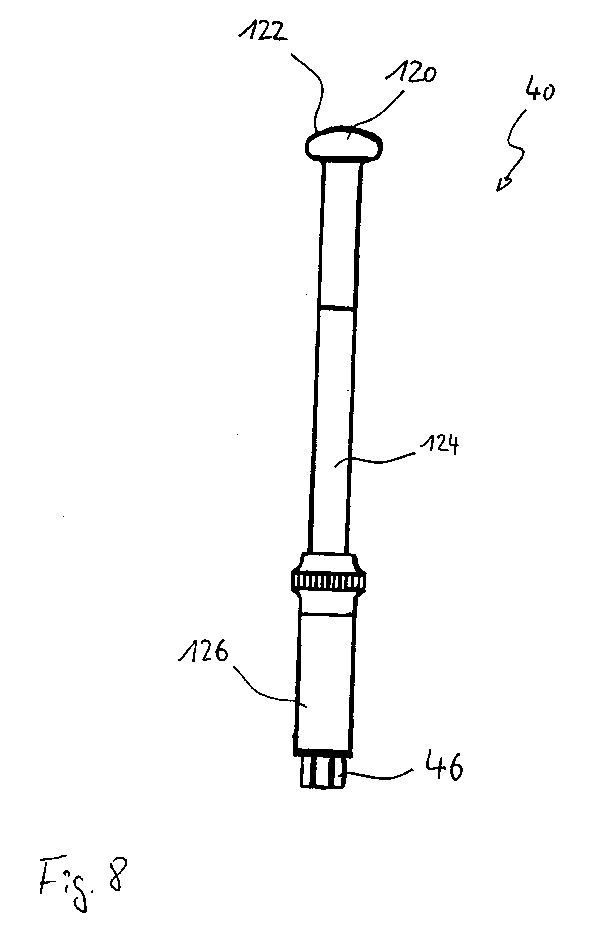

In Fig. 8 ist ein zweites Ausführungsbeispiel einer erfindungsgemäßen Eintreibvorrichtung 40 zum Eintreiben eines Implantats lateral in einen Knochendeckel oder ein Knochenfragment dargestellt. Die Eintreibvorrichtung 40 weist eine längliche Bauform auf und besitzt eine Aufnahme 46 für das Implantat sowie eine knaufartige Krafteinleitungsstruktur 120 mit einer sphärisch gekrümmten Krafteinleitungsfläche 122. Die Eintreibvorrichtung 40 umfasst weiterhin einen im wesentlichen zylindrischen Körper 124, welcher kraftübertragend zwischen der Krafteinleitungsstruktur 120 und der Aufnahme 46 angeordnet ist. An seinem der Aufnahme 46 zugewandten Ende trägt der zylindrische Körper 124 ein Handstück 126, welches den zylindrischen Körper 124 radial außen umgibt. Das Handstück 126 ist in axialer Richtung unbeweglich mit dem Körper 124 gekoppelt. Die Aufnahme 46 besitzt den in den Fig. 7a und 7b dargestellten Selbsthaltemechanismus für das Implantat.

Zur Befestigung des Implantats an z.B. einer Knochenplatte wird dieses zunächst mittels des Selbsthaltemechanismus im Bereich der Aufnahme 46 fixiert. Anschließend wird das Implantat wie vorstehend beschrieben positioniert und eine Schlagkraft beispielsweise mittels eines Hammers in die Krafteinleitungsfläche 122 der Krafteinleitungsstruktur 120 eingeleitet. Die Schlagkraft überträgt sich von der Krafteinleitungsstruktur über den Körper 124 und die Aufnahme 46 auf das Implantat 10, welches daraufhin lateral in den Knochendeckel eingetrieben wird.

Fig. 9 zeigt einen Knochendeckel 130, welcher eine obere Knochentafel 132 (erste kortikale Schicht), eine untere Knochentafel 134 (zweite kortikale Schicht) sowie einen zwischen der oberen Knochentafel 132 und der unteren Knochentafel 134 angeordneten, spongiösen Bereich 136 besitzt. Das Implantat 10 wurde mittels der unter Bezugnahme auf die Fig. 4 bis 7b beschriebenen Eintreibvorrichtung 40 am Knochendeckel 130 befestigt. Die Spitze 16 des Implantats 10 wurde so tief in den spongiösen Bereich 136 des Knochendeckels 130 eingetrieben, dass der Fortsatz 14 lateral am Knochendeckel 130 anliegt.

Nach dem Befestigen einer Mehrzahl von Implantaten 10 am Knochendeckel 130 wurde der Knochendeckel in der entsprechenden Öffnung des Schädelknochens 140 positioniert. Dabei gelangt die Unterseite des Auflagearms 22 des Implantats 10 in Anlage mit der oberen Knochentafel 142 des Schädelknochens 140. Der Schädelknochen 140 besitzt ebenfalls eine spongiösen Bereich 146, welcher zwischen der oberen Knochentafel 142 und einer unteren Knochentafel 144 angeordnet ist.

Nach dem Positionieren des mit dem Implantat 10 versehenen Knochendeckels 130 in der Öffnung des Schädelknochens 140 muss der Knochendeckel 130 innerhalb der Öffnung des Schädelknochens 140 festgelegt werden. Zu diesem Zweck werden die Implantate 10 mittels Knochenschrauben 150 am Schädelknochen 140 befestigt. Dabei wird der sphärische Kopf 152 jeder Knochenschraube 150 vollständig im Schraubenloch des Implantats 10 versenkt.

Bei bikortikalen Knochen wie dem in Fig. 9 dargestellten Knochendeckel 130 ist es zweckmäßig, die Spitze 16 des Implantats 10 in den zwischen den beiden kortikalen Schichten angeordneten spongiösen Bereich einzutreiben. Das Eintreiben der Spitze 16 in eine kortikale Schicht ist jedoch ebenfalls möglich und bei monokortikalen Knochen sogar unabdingbar.

## Patentansprüche

1. Vorrichtung (40) zum Befestigen eines selbsthaltenden Implantats (10) an einem Knochendeckel (130) oder einem Knochenfragment, mit:
- einer Aufnahme (46) für das Implantat (10);
- einem mit der Aufnahme (46) koppelbaren Eintreibmechanismus (56) zum Eintreiben des Implantats lateral in den Knochendeckel oder das Knochenfragment, der einen ersten Schlitten (58) aufweist, der entlang eines ersten Führungsbolzens (60) koaxial zur Aufnahme (46) geführt wird und gegen eine Federkraft verschiebbar ausgebildet ist, um eine Schlagkraft auf die Aufnahme (46) auszuüben; und
- einem Betätigungsmechanismus (54) für den Eintreibmechanismus (56),
**dadurch gekennzeichnet, dass** der Eintreibmechanismus (56) weiter aufweist:
- einen zweiten Schlitten (72), der ebenfalls gegen eine Federkraft entlang eines zweiten Führungsbolzens (70) verschiebbar ist, wobei der zweite Führungsbolzen (70) sich parallel zu dem ersten Führungsbolzen (60) erstreckt,
- eine Koppeleinrichtung (84) zum Koppeln des Betätigungsmechanismus (54) mit dem ersten Schlitten (58), wobei der Betätigungsmechanismus (54) mit dem zweiten Schlitten (72) gekoppelt ist, so dass die Koppeleinrichtung (84) ein Koppeln des ersten Schlittens (58) mit dem zweiten"Schlitten(72) ermöglicht, und wobei die Koppeleinrichtung (84) dazu ausgebildet ist, den ersten Schlitten (58) bei Betätigung des Betätigungsmechanismus entgegen einer Federkarft (64) vorzuspannen, und
- eine Entkoppeleinrichtung (92) zum Entkoppeln des ersten Schlittens (58) von dem zweiten Schlitten (72) so das der vorgespannte erste Schlitten (58) entlang des ersten Führungsbolzens (60) beschleunigt wird und eine Schlagkraft auf die Aufnahme ausübt.

2. Vorrichtung nach Anspruch 1,
**dadurch gekennzeichnet, dass** die Schlagkraft mittels des Eintreibmechänismus (56) auf die Aufnahme (46) ausübbar ist.

3. Vorrichtung nach einem der Ansprüche 1 oder 2,
**dadurch gekennzeichnet, dass** der Betätigungsmechanismus (54) zum Auslösen des Koppelns und Entkoppelns vorzugsweise fingerbetätigbar ausgebildet ist.

4. Vorrichtung nach Anspruch 3,
**dadurch gekennzeichnet dass** die Koppeleinrichtung (84) vorgespannt ist, um den Betätigungsmechanismus (54) mit dem ersten Schlitten (58) zu koppeln.

5. Vorrichtung nach einem der Ansprüche 1 bis 4,
**dadurch gekennzeichnet, dass** die Vorrichtung als Pistole (40) ausgestaltet ist.

6. Vorrichtung nach Anspruch 5,
**dadurch gekennzeichnet, dass** der Betätigungsmechanismus als Pistolenabzug (54) ausgebildet ist.

7. Vorrichtung nach Anspruch 6,
**dadurch gekennzeichnet, dass** der Pistolenabzug (54) mit dem zweiten Schlitten (72) gekoppelt ist.

8. Vorrichtung nach einem der Ansprüche 5 bis 7,
**dadurch gekennzeichnet, dass** die Aufnahme (46) nach Art eines Pistolenlaufs (44) bezüglich eines Pistolenkörpers (42) der Pistole (40) angeordnet ist.

9. Vorrichtung nach einem der vorstehenden Ansprüche,
**dadurch gekennzeichnet, dass** mindestens eine Spitze des Implantats (10) lateral in den Knochendeckel (130) oder das Knochenfragment eintreibbar ist.

10. Vorrichtung nach einem der Ansprüche 1 bis 9,
**dadurch gekennzeichnet, dass** die Aufnahme (46) einen Selbsthaltemechanismus (110) für das Implantat (10) umfasst.

11. System, umfassend
ein selbsthaltendes Implantat (10) zum Festlegen eines Knochendeckels (130) oder eines Knochenfragments, mit
- einem Auflageelement (12) mit einer Oberseite (18) und einer dem Knochendeckel (130) oder dem Knochenfragment zugewandeten Unterseite (20) und
- einem an der Unterseite (20) des Auflageelements (12) angeordneten Fortsatz (14), der mindestens eine, sich in Richtung auf den Knochendeckel (130) oder das Knochenfragment erstreckende Spitze (16) trägt, die zur selbsthaltenden Verankerung des Implantats lateral in den Knochendeckel (130) oder das Knochenfragment eintreibbar ist,
**dadurch gekennzeichnet, dass** das Auflageelement (12) zwei sich in entgegengesetzte Richtungen erstreckende Auflagearme (22, 24) umfasst, wobei ein erster der beiden Auflagearme (22) mit einem Schädelknochen (140) und ein zweiter der beiden Auflagearme (24) mit dem Knochendeckel (130) oder dem Knochenfragment zusammenwirken kann,
und ferner **gekennzeichnet durch** eine Vorrichtung nach einem der Ansprüche 1 bis 10.

## Claims

1. A device (40) for securing a self-retaining implant (10) to a bone cover (130) or a bone fragment, with:
- a receiving element (46) for the implant (10);
- a driving-in mechanism (56) which can be coupled with the receiving element (46) for driving the implant laterally into the bone cover or bone fragment, comprising a first carriage (58) being guided coaxial to the receiving element (46) along a first guide bolt (60) and adapted to be displaceable against a spring force in order to apply a striking force to the receiving element (46); and
- an operating mechanism (54) for the driving-in mechanism (56),
**characterized in that** the driving-in mechanism (56) further comprises:
- a second carriage (72) being also displaceable against a spring force along a second guide bolt (70), wherein the second guide bolt (70) extends parallel to the first guide bolt (60),
- a coupling device (84) for coupling of the operating mechanism (54) with the first carriage (58), wherein the operating mechanism (54) is coupled with the second carriage (72) such that the coupling device (84) allows a coupling of the first carriage (58) with the second carriage (72), and wherein the coupling device (84) is adapted to bias the first carriage (58) against a spring force (64) when the operating mechanism is operated; and
- a decoupling device (92) for decoupling the first carriage (58) from the second carriage (72) such that the biased first carriage (58) is accelerated along the first guide bolt (60) and applies a striking force to the receiving element.

2. The device according to claim 1,
**characterized in that** the striking force can be applied to the receiving element (46) by means of the driving-in mechanism (56).

3. The device according to claim 1 or claim 2,
**characterized in that** the operating mechanism (54) for activating the coupling or decoupling is preferably adapted to be finger-operated.

4. The device according to claim 3,
**characterized in that** the coupling device (84) is biased in order to couple the operating mechanism (54) with the first carriage (58).

5. Device according to any of claims 1 to 4,
**characterized in that** the device is constructed as a pistol (40).

6. Device according to claim 5,
**characterized in that** the operating mechanism is constructed as a pistol trigger (54).

7. Device according to claim 6,
**characterized in that** the pistol trigger (54) is coupled with the second carriage (72).

8. The device according to any of claims 5 to 7,
**characterized in that** the receiving element (46) is arranged in the manner of a pistol barrel (54) in relation to a pistol body (42) of the pistol (40).

9. Device according to any of the forgoing claims,
**characterized in that** at least one spike of the implant (10) can be driven laterally into the bone cover (13) or into the bone fragment.

10. Device according to any of claims 1 to 9,
**characterized in that** the receiving element (46) comprises a self-locking mechanism (110) for the implant (10).

11. System comprising
a self-retaining implant (10) for attaching a bone cover (130) or a bone fragment, with:
- a support element (12) with an upper side (18) and a lower side (20) which faces the bone cover (130) or the bone fragment, and
- an extension (14) which is arranged on the lower side (20) of the support element (12), the extension (14) supporting at least one spike (16) that extends towards the bone cover (130) or the bone fragment and can be driven laterally into the bone cover (130) or bone fragment for anchoring the implant in self-retaining fashion,
**characterized in that** the support element (12) comprises two support arms (22, 24) extending in opposite directions, the first of the two support arms (22) being adapted to cooperate with a skull bone (140) and the second of the two support arms (24) being adapted to cooperate with the bone cover (130) or the bone fragment, and further **characterized by** a device according to any of claims 1 to 10.

## Revendications

1. Dispositif (40) pour la fixation d'un implant auto-rétentif (10) sur un volet osseux (130) ou d'un fragment osseux, avec :
- un logement (46) pour l'implant (10) ;
- un mécanisme d'enfoncement (56) pouvant être accouplé au logement (46) et servant à enfoncer latéralement l'implant dans le volet osseux ou le fragment osseux, lequel mécanisme présente un premier chariot (58) guidé coaxialement au logement (46) le long d'une première broche de guidage (60) et conçu mobile en translation contre une force de ressort pour exercer une force de frappe sur le logement (46) ; et
- un mécanisme d'actionnement (54) pour le mécanisme d'enfoncement (56),
**caractérisé en ce que** le mécanisme d'enfoncement (56) présente en outre :
- un second chariot (72) qui est lui aussi conçu mobile en translation le long d'une seconde broche de guidage (70) contre une force de ressort, la seconde broche de guidage (70) s'étendant parallèlement à la première broche de guidage (60),
- un dispositif d'accouplement (84) pour accoupler le mécanisme d'actionnement (54) au premier chariot (58), le mécanisme d'actionnement (54) étant accouplé au second chariot (72) si bien que le dispositif d'accouplement (84) permet l'accouplement du premier chariot (58) au second chariot (72), et le dispositif d'accouplement (84) étant conçu pour pré-contraindre le premier chariot (58) contre une force de ressort (64) lorsqu'est actionné le mécanisme d'actionnement, et
- un dispositif de désaccouplement (92) pour désaccoupler le premier chariot (58) du second chariot (72) si bien que le premier chariot (58) précontraint est accéléré le long de la première broche de guidage (60) et une force de frappe est exercée sur le logement.

2. Dispositif selon la revendication 1,
**caractérisé en ce que** la force de frappe peut être exercée au moyen du mécanisme d'enfoncement (56) sur le logement (46).

3. Dispositif selon la revendication 1 ou 2,
**caractérisé en ce que**, pour déclencher l'accouplement et le désaccouplement, le mécanisme d'actionnement (54) est conçu de préférence actionnable au doigt.

4. Dispositif selon la revendication 3,
**caractérisé en ce que** le dispositif d'accouplement (84) est précontraint pour accoupler le mécanisme d'actionnement (54) au premier chariot (58).

5. Dispositif selon l'une des revendications 1 à 4,
**caractérisé en ce que** le dispositif est conçu comme un pistolet (40).

6. Dispositif selon la revendication 5,
**caractérisé en ce que** le mécanisme d'actionnement est conçu comme une gâchette de pistolet (54).

7. Dispositif selon la revendication 6,
**caractérisé en ce que** la gâchette de pistolet (54) est accouplée au second chariot (72).

8. Dispositif selon l'une des revendications 5 à 7,
**caractérisé en ce que** le logement (46) est monté à la manière d'un canon de pistolet (44) par rapport au corps (42) du pistolet (40).

9. Dispositif selon l'une des revendications précédentes,
**caractérisé en ce qu'**au moins une pointe de l'implant (10) peut être enfoncée latéralement dans le volet osseux (130) ou le fragment osseux.

10. Dispositif selon l'une des revendications 1 à 9,
**caractérisé en ce que** le logement (46) comprend un mécanisme d'auto-retenue (110) pour l'implant (10).

11. Système, comportant
un implant auto-rétentif (10) pour la fixation d'un volet osseux (130) ou d'un fragment osseux, avec
- un élément d'appui (12) avec une face supérieure (18) et une face inférieure (20) dirigée vers le volet osseux (130) ou le fragment osseux et
- un prolongement (14) disposé sur la face inférieure (20) de l'élément d'appui (12) qui porte au moins une pointe (16) s'étendant en direction du volet osseux (130) ou du fragment osseux et pouvant être enfoncée latéralement dans le volet osseux (130) ou le fragment osseux pour l'ancrage auto-rétentif de l'implant,
**caractérisé en ce que** l'élément d'appui (12) comprend deux bras d'appui (22, 24) s'étendant dans des directions opposées, un premier des deux bras d'appui (22) pouvant coopérer avec un os crânien (140) et un second des deux bras (24) pouvant coopérer avec le volet osseux (130) ou le fragment osseux,
et **caractérisé de plus par** un dispositif selon l'une des revendications 1 à 10.
